# EUROPEAN PATENT APPLICATION

(11) **EP 4 357 779 A1**
(43) Date of publication of application: **24.04.2024**
(21) Application number: 22202920.9
(22) Date of filing: 21.10.2022
(51) Int. Cl.: G01N 33/569, C12Q 1/689

(54) **INFLAMMATORY DISORDER IN A FELIDAE**

(71) Applicant: Mars, Incorporated, McLean, VA 22101 (US)
(72) Inventor: LANGON, Xavier, 30470 AIMARGUES (FR)
(74) Representative: Cabinet Nony

(57) **Abstract**

The present invention relates to an *in vitro* method for the diagnosis or follow-up of an inflammatory disorder in a Felidae. It also relates to compounds, and compositions thereof, for treating an inflammatory disorder in a Felidae.

## Description

### FIELD OF THE INVENTION

The present invention relates to the field of diagnosis and follow-up of an inflammatory disorder in a Felidae, in particular of a pet animal such as a cat.

More particularly, the present invention relates to the field of diagnosis and follow-up of Feline atopic syndrome (FAS), which includes Feline atopic skin syndrome (FASS), Flea allergy dermatitis (FAD), Feline food allergy (FFA), and associated conditions.

The present invention further relates to compounds, and compositions thereof, for treating an inflammatory disorder in a Felidae.

### BACKGROUND OF THE INVENTION

The complex intestinal microbiome is widely investigated in humans since the last fifty years. Studies have shown that the microbiome can be affected by different environmental factors and diseases, including systemic inflammatory diseases, with major or minor effects. In humans, long standing diseases such as obesity, inflammatory bowel diseases, allergic rhinitis, and atopic dermatitis are reportedly linked with the microbiome, in a controversial causal manner. Current studies focus in understanding its preventive role during infancy and bacterial strains involved in adults with allergic status.

In particular, atopic dermatitis is a chronic inflammatory skin condition, starting in early childhood and is usually the first manifestation of the atopic march. Maturation of an inappropriate T-helper type 2 (Th2) immune response that predominates at birth to a Th1 predominance in infancy and adulthood is conditional on the presence of commensal gut bacteria. The microbiome has a well-documented role in human atopic dermatitis, in particular the association of dysbiosis and an increase risk of atopy.

American adults with allergies, especially to nuts and seasonal pollen, have low diversity, reduced Clostridiales, and increased Bacteroidales in their gut microbiota. This dysbiosis might be targeted to improve treatment or prevention of allergy **(**Hua et al ; « Allergy associations with the adult fecal microbiota : Analysis of the American Gut Projet » ; EBioMedicine, 2016).

Under certain conditions, it is hypothesized that this gut homeostasis condition is also disrupted in other non-human mammals. For example, dysbiosis may occur with a reduction of bacterial diversity, a loss of beneficial bacteria and an overgrowth of pathogens. Because microbial shifts are often noted with disease, it is thought that microbiome profiles may be used as biomarkers for diagnosis and/or monitoring in the future, as reported in the fecal microbiome of dogs **(**Lin et al.; « An ambient temperature collection and stabilization strategy for canine microbiota studies » ; Nature, 2020).

Inflammatory disorders, in particular those resulting from feline allergic diseases, are prevalent in cats **(**Halliwell et al. ; « Feline allergic diseases : introduction and proposed nomenclature » ; Veterinary Dermatology, 2021). Feline atopic skin syndrome (FASS) is an inflammatory and pruritic skin syndrome of cats manifested by a spectrum of reaction patterns, that may be associated with IgE antibodies to environmental allergens **(**Richard Halliwell; « Immunopathogenesis of the feline atopic syndrome » ; Veterinary Dermatology, 2021).

The cutaneous lesions of FASS are variable in appearance, and include miliary dermatitis (MD), self-inflicted alopecia/hypotrichosis (SIAH), head and neck pruritus (HNP) and eosinophilic granuloma complex (EGC) **(**Santoro et al. ; « Clinical signs and diagnosis of feline atopic syndrome: detailed guidelines for a correct diagnosis » ; Veterinary. Dermatology, 2021).

In Felidae, the new consideration of gut microbiota role and the fast development of next-generation sequencing have also given access to these microbial data **(**Lyu et al.; "Past, Present, and Future of Gastrointestinal Microbiota Research in Cats"; Front. Microbiol., 2020). Similarly to other mammals, the vast majority of the gastrointestinal (GI) microbiota in cats (over 99%) is composed of the predominant bacterial phyla Firmicutes, Bacteroidetes, Actinobacteria, and Proteobacteria.

One study has demonstrated a correlation between Feline Chronic Enteropathy severity and an increased Enterobacteriaceae **(**Simpson; Chapter 10 - The Role of the Microbiota in Feline Inflammatory Bowel Disease; August's Consultations in Feline internal medicine, Vol. 7, 2016).

In one study, an increase of Bifidobacteriales and Lactobacilliacee was further associated with feline immunodeficiency virus infection **(**Weese et al. "The rectal microbiota of cats infected with feline immunodeficiency virus infection and uninfected controls"; Veterinary Microbiology, 2015).

There is thus a need for new biomarkers for detecting inflammatory disorders in Felidae; and particularly in cats.

There is also a need for compounds and compositions for treating such inflammatory disorders in non-human mammals, including Felidae.

The invention has for purpose to meet the above-mentioned needs.

### SUMMARY OF THE INVENTION

A first main aspect of the present invention relates to an *in vitro* method for the diagnosis or follow-up of an inflammatory disorder in a Felidae, comprising a step of determining the level of occurrence of *Bacteroides vulgatus* **or** *Helicobacter canis* in a digestive tract sample thereof; wherein:
- an increase of the level of *Bacteroides vulgatus* is indicative of the occurrence of the inflammatory disorder, and/or
- a decrease of the level of *Helicobacter canis* is indicative of the occurrence of the inflammatory disorder, and/or
- an increase of the ratio level of *Bacteroides vulgatus* over *Helicobacter canis,* is/are indicative of the inflammatory disorder.

A second main aspect of the present invention relates to a compound, or composition thereof, selected from the list consisting of:
- a corticosteroid, in particular a glucocorticoid or a mineralocorticosteroid;
- ciclosporin;
- oclatinib;
- H1-receptor blocking antihistamine ;
- essential fatty acid selected from the group consisting of: linoleic acid, linolenic acid and arachidonic acid ;
- palmitoylethanolamide (PEA)
- and combinations thereof;
for use in a method for treating an inflammatory disorder in a Felidae, said Felidae being characterized by: an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract.

### DETAILED DESCRIPTION OF THE INVENTION

Bacteria species *Bacteroides vulgatus* and *Helicobacter canis* are known to be present in the gut microbiota of Felidae, in particular domestic cats.

Yet, to the best of their knowledge, the inventors are the first to report that those two species exhibit a difference in prevalence, namely *Bacteroides vulgatus* and *Helicobacter canis,* in a group of cats with feline atopic skin syndrome (FASS). This modulation was unexpected, due to the lack of studies on the relationship between the gut microbiota and inflammatory disorders, such as FASS, in Felidae.

Accordingly, the inventors report :
- an increase of the level of *Bacteroides vulgatus* (e.g. over a reference value, for example healthy individuals), which is indicative of the occurrence of the inflammatory disorder, and/or
- a decrease of the level of *Helicobacter canis* (e.g. below a reference value, for example healthy individuals), which is indicative of the occurrence of the inflammatory disorder, and/or
- an increase of the ratio level of *Bacteroides vulgatus* over *Helicobacter canis* (e.g. when compared to a healthy individual or to the same individual in a previous/healthier condition), which is/are indicative of the occurrence of the inflammatory disorder.

In particular, the modulation of *Helicobacter canis,* which is less preponderant in the FASS group, is unexpected because this bacterial strain was previously described in both diarrheic and healthy patients in other studies, including Foley et al. (« Isolation of Helicobacteri canis from a Colony of bengal Cats with Endemic Diarrhea » ; Journal of Clinical Microbiology; 1999) and Stanley et al. (« helicobacter canis sp. nov., a new species from dogs : an integrated study of phenotype and genotype » ; J. Gen. Microbiol. 1993).

Without wishing to be bound by the theory, the inventors are of the opinion that the above-mentioned modulations constitute a novel biomarker of inflammatory disorders in Felidae, including their associated symptoms with Feline atopic syndrome (FAS) and feline atopic skin syndrome (FASS).

The inventors further disclose compounds and compositions, for treating inflammatory disorders toward sub-populations of Felidae, characterized by :
- an increase of the level of *Bacteroides vulgatus* over a reference value, and/or
- a decrease of the level of *Helicobacter canis* below a reference value, and/or
- an increase of the ratio level of *Bacteroides vulgatus* over *Helicobacter canis.*

In particular, the inventors further disclose compounds and compositions, for treating inflammatory disorders toward sub-populations of Felidae, characterized by :
- an increase of the level of *Bacteroides vulgatus* over a reference value, and/or
- a decrease of the level of *Helicobacter canis* below a reference value, and/or
- an increase of the ratio level of *Bacteroides vulgatus* over *Helicobacter canis.*

More particularly, the inventors disclose such compounds and compositions, previously reported as efficient toward the treatment of an allergic disorder, an inflammatory skin disorder, an inflammatory tract disorder, and/or a respiratory tract disorder, and preferably of a Feline Atopic Syndrome (FAS), which is considered as particularly relevant for the identified sub-population(s) of Felidae.

According to a **first main embodiment,** the invention thus relates to an *in vitro* method for the diagnosis or follow-up of an inflammatory disorder in a Felidae, comprising a step of determining the level of occurrence of *Bacteroides vulgatus* or *Helicobacter canis* in a digestive tract sample thereof; wherein:
- an increase of the level of *Bacteroides vulgatus* is indicative of the occurrence of the inflammatory disorder, and/or
- a decrease of the level of *Helicobacter canis* is indicative of the occurrence of the inflammatory disorder, and/or
- an increase of the ratio level of *Bacteroides vulgatus* over *Helicobacter canis,* is/are indicative of the inflammatory disorder.

According to a particular embodiment, the *in vitro* method comprises a step of determining the level of occurrence of *Bacteroides vulgatus* in the digestive tract sample thereof.

According to a particular embodiment, the *in vitro* method comprises a step of determining the level of occurrence of *Helicobacter canis* in the digestive tract sample thereof.

According to a particular embodiment, the *in vitro* method comprises a step of determining the level of occurrence of *Bacteroides vulgatus* and *Helicobacter canis* in the digestive tract sample thereof.

According to a particular embodiment of the *in vitro* method, the inflammatory disorder is selected from a list consisting of: an allergic disorder, an inflammatory skin disorder, an inflammatory digestive tract disorder, and a respiratory tract disorder.

According to a particular embodiment of the *in vitro* method, the inflammatory disorder is Feline atopic syndrome (FAS).

According to a particular embodiment of the *in vitro* method, the inflammatory disorder is selected from: Feline atopic skin syndrome (FASS), Flea allergy dermatitis (FAD), Feline food allergy (FFA).

According to a particular embodiment of the *in vitro* method, the inflammatory disorder is Feline atopic skin syndrome (FASS).

According to a particular embodiment of the *in vitro* method, the inflammatory disorder is selected from: miliary dermatitis (MD), self-inflicted alopecia/hypotrichosis (SIAH), head and neck pruritus (HNP), eosinophilic granuloma complex (EGC).

According to a particular embodiment of the *in vitro* method, the digestive tract sample is selected from a list consisting of: a fecal sample, a gastric sample, a saliva sample, or fractions thereof; in particular a fecal sample or fractions thereof.

According to a particular embodiment of the *in vitro* method, the Felidae is selected from a list consisting of: cheetah, puma, jaguar, leopard, lion, lynx, liger, tiger, panther, bobcat, ocelot, smilodon, caracal, serval and cats; in particular wild cats and domestic cats, including breeds and hybrids thereof; and most preferably domestic cats.

According to a particular embodiment of the *in vitro* method, the Felidae is selected from a list consisting of: Abyssinian, Aegean, American Bobtail, American Curl, American Ringtail, American Shorthair, American Wirehair, Aphrodite Giant, Arabian Mau, Asian, Asian Semi-longhair, Australian Mist, Balinese, Bambino, Bengal, Birman, Bombay, Brazilian Shorthair, British Longhair, British Shorthair, Burmese, Burmilla, California Spangled, Chantilly-Tiffany, Chartreux, Chausie, Colorpoint Shorthair, Cornish Rex, Cymric, Manx Longhair, Long-haired Manx, Cyprus, Devon Rex, Donskoy, Don Sphynx, Dragon Li, Chinese Li Hua, Dwelf, Egyptian Mau, European Shorthair, Exotic Shorthair, Foldex, German Rex, Havana Brown, Highlander, Himalayan, Colorpoint Persian, Japanese Bobtail, Javanese, Colorpoint Longhair, Kanaani, Khao Manee, Kinkalow, Korat, Korean Bobtail, Korn Ja, Kurilian Bobtail, Kuril Islands Bobtail, Lambkin, LaPerm, Lykoi, Maine Coon, Manx, Mekong Bobtail, Minskin, Minuet, Munchkin, Nebelung, Norwegian Forest Cat, Ocicat, Ojos Azules, Oregon Rex, Oriental Bicolor, Oriental Longhair, Oriental Shorthair, Persian, Peterbald, Pixie-bob, Ragamuffin, Liebling, Ragdoll, Raas, Russian Blue, Russian White, Russian Black, Russian Tabby, Sam Sawet, Savannah, Scottish Fold, Selkirk Rex, Serengeti, Serrade Petit, Siamese, Siberian, Siberian Forest Cat, Neva Masquerade, Singapura, Snowshoe, Sokoke, Somali, Sphynx, Suphalak, Thai, Thai Lilac,Thai Blue Point, Thai Lilac Point, Tonkinese, Toybob, Toyger, Turkish Angora, Turkish Van, Turkish Vankedisi, Ukrainian Levkoy, York Chocolate.

According to a particular embodiment, the *in vitro* method comprises a step of determining the level of occurrence of *Bacteroides vulgatus* or *Helicobacter canis* comprising determining, preferably quantifying, all or part of a nucleic acid sequence of said *Bacteroides vulgatus* or *Helicobacter canis.*

According to some particular embodiments of the *in vitro* method for the diagnosis or follow-up of an inflammatory disorder, the method comprises a step of contacting a digestive tract sample, or a fraction thereof, e.g. a feces/stool sample, with one or more probes capable of specifically hybridizing to one or more gut microbiome nucleic acid sequences, in particular *Bacteroides vulgatus* and/or *Helicobacter canis.*

According to some particular embodiments of the *in vitro* method for the diagnosis or follow-up of an inflammatory disorder, the method comprises a step of detecting and/or isolating the probes that specifically hybridize to gut microbiome nucleic acid sequences within the sample.

According to some particular embodiments of the *in vitro* method for the diagnosis or follow-up of an inflammatory disorder, the method comprises a step of sequencing gut microbiome nucleic acid sequences which are susceptible to be present in the digestive tract sample.

According to some particular embodiments of the *in vitro* method for the diagnosis or follow-up of an inflammatory disorder, the method comprises a step of comparing gut microbiome nucleic acid sequences with a database comprising reference gut microbiome nucleic acid sequences, in particular reference gut microbiome nucleic acid sequences from *Bacteroides vulgatus* and/or *Helicobacter canis.*

According to some particular embodiments of the *in vitro* method for the diagnosis or follow-up of an inflammatory disorder, the method comprises the following steps:
a) contacting a digestive tract sample, or a fraction thereof, with one or more probes capable of specifically hybridizing to one or more gut microbiome nucleic acid sequences, in particular *Bacteroides vulgatus* and/or *Helicobacter canis;*
b) optionally detecting and/or isolating the probes that specifically hybridize to gut microbiome nucleic acid sequences within the sample;
c) sequencing the gut microbiome nucleic acid sequences hybridized to the one or more probes; and
d) comparing the sequences from (c) with a database comprising reference gut microbiome nucleic acid sequences, in particular reference gut microbiome nucleic acid sequences from *Bacteroides vulgatus* and/or *Helicobacter canis,* in order to determine the identity of the hybridized gut microbiome nucleic acid in the sample.

According to a **second main embodiment,** the invention relates to a compound, or composition thereof, for example a food composition, for use in a method for treating an inflammatory disorder in a Felidae, said Felidae being characterized by: an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract.

According to **said second main embodiment,** the invention relates to a compound, or composition thereof, selected from the list consisting of:
- a corticosteroid, in particular a glucocorticoid or a mineralocorticosteroid;
- ciclosporin;
- oclatinib;
- H1-receptor blocking antihistamine ;
- essential fatty acid selected from the group consisting of: linoleic acid, linolenic acid and arachidonic acid ;
- palmitoylethanolamide (PEA), for example ultra-micronized PEA ;
- and combinations thereof;
for use in a method for treating an inflammatory disorder in a Felidae, said Felidae being characterized by: an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract.

According to particular embodiments, the compound, or composition thereof, for use according the present disclosure, is a food composition, which may or may not be nutritionally complete; for example a food additive.

According to particular embodiments, the compound, or composition thereof, for use according the present disclosure, is for use in a method for treating an inflammatory disorder in a Felidae, said inflammatory disorder being Feline atopic skin syndrome (FASS).

The invention also relates to a method for treating an inflammatory disorder in a Felidae, said Felidae being characterized by: an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract.

The invention also related to a method for treating an inflammatory disorder in the Felidae, comprising the step of administering to the felidae at least one selected from: a corticosteroid, in particular a glucocorticoid or a mineralocorticosteroid, ciclosporin, oclatinib, H1-receptor blocking antihistamine, essential fatty acid selected from the group consisting of: linoleic acid, linolenic acid and arachidonic acid, palmitoylethanolamide (PEA), and/or combinations thereof.

The invention also relates to a method for treating an inflammatory disorder in a Felidae, comprising the steps of:
a) determining an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract of the felidae, in particular in a digestive tract sample of the felidae;
b) administering to the felidae at least one selected from: a corticosteroid, in particular a glucocorticoid or a mineralocorticosteroid, ciclosporin, oclatinib,H1-receptor blocking antihistamine, essential fatty acid selected from the group consisting of: linoleic acid, linolenic acid and arachidonic acid, palmitoylethanolamide (PEAum), and/or combinations thereof.

### Definitions

As used herein, the term *"inflammatory disease"* is meant to refer to any disease/disorder associated with inflammation, without restrictions, including those associated with one selected from an allergic (or atopic) disorder, an inflammatory skin disorder, an inflammatory digestive tract disorder, and a respiratory tract disorder. For example, the term may encompass one or more of the following conditions and syndromes: an atopic disease, Feline Atopic Syndrome (FAS), Feline Atopic Skin Syndrome (FASS), Feline asthma, Feline food allergy (FFA). The term further encompasses allergic dermatitis, allergic enteritis and asthma.

As used herein, the term *"atopy"* or *"atopic disease"* is meant to refer to the commonly acknowledged definition, without restrictions. The essential features of an atopic disease are defined as a predisposition to allergic disease affecting the skin. Hence, the term encompasses any clinical dermatitis hypersensitivity associated with an inherited tendency to produce immunoglobulin E (IgE) antibodies in response to environmental proteins such as pollen, house dust mite, and food allergens.

As used herein, the term *"Feline Atopic Syndrome"* or *"FAS"* is meant to encompass allergic diseases of the skin, gastrointestinal tract and respiratory tract, in the broadest sense, as described in Halliwell et al. (« Feline allergic diseases : introduction and proposed nomenclature » ; Veterinary Dermatology, 2021). Hence, the term encompasses allergic dermatitis associated with environmental allergen, food allergy and asthma that may be associated with IgE antibodies. Accordingly, the term encompasses those inflammatory diseases selected from: Feline atopic skin syndrome (FASS), Flea allergy dermatitis (FAD), Feline food allergy (FFA) and Feline asthma.

As used herein, the term *"Feline Atopic Skin Syndrome"* or *"FASS"* is meant to refer to the commonly acknowledged definition, without restrictions. Hence, the term encompasses allergic skin diseases, associated with environmental allergies. More particularly it may encompass one or more of the following conditions: miliary dermatitis (MD), self-inflicted alopecia/hypotrichosis (SIAH), head and neck pruritus (HNP), eosinophilic granuloma complex (EGC). In particular, FASS is commonly associated with an inflammatory and pruritic skin syndrome (e.g. found in cats). Food allergy anf flea allergy can both contribute to the syndrome.

As used herein, the term *"Feline asthma"* is meant to refer to the commonly acknowledged definition, without restrictions. Hence, the term encompasses an eosinophilic inflammatory disease affecting the bronchioles and leading to spontaneous reversible bronchoconstriction and airway remodelling, manifested by acute respiratory distress or chronic coughing and expiratory wheezing, that may be associated with IgE antibodies to inhaled allergens.

As used herein, the term *"Feline food allergy"* is meant to refer to the commonly acknowledged definition, without restrictions. Hence, the term encompasses the clinical manifestations, including those of FASS, that are attributable to the allergen-specific reactivity to an ingested food item.

As used herein, the term *"corticosteroid'* is meant to refer to any corticosteroid hormone without restrictions, including naturally-occuring corticosteroids and synthetic corticosteroids. Hence, the term encompasses, in particular, a glucocorticoid or a mineralocorticosteroid, without limitation to a particular administration route. Hence, the term encompasses topical, inhaled and system corticosteroids (e.g. glucocorticoids) in the absence of indication of the contrary.

As used herein, the term *"glucocorticorticoid'* is meant to refer to a steroid capable of binding to the glucocorticoid receptor without restrictions. Such compounds encompass to, but are not limited to, dexamethasone, dexamethasone containing agents, cortivazol, hydrocortisone, methylprednisolone, paramethasone, prednisone, prednisolone, prednylidene, cortisone, budesonide, betamethasone, beclomethasone, mometasone furoate, fluticasone, flunisolide, Triamcinolone Acetonide, methylprednisone, derivatives and salts thereof. Other particular examples of glucocorticoids include dexamethasone base, dexamethasone sodium phosphate, dexamethasone hemi succinate, dexamethasone sodium succinate, dexamethasone succinate, and dexamethasone acetate.

As used herein, the term « *ciclosporin* », or « *cyclosporine* » or « *cyclosporin* » or « *cyclosporin A* », is meant to refer to the compound referenced under CAS number 59865-13-3, and salts thereof; which thus may include microemulsion and liquid forms thereof.

As used herein, the term « *oclatinib* » is meant to refer to the compound referenced under CAS number 1208319-26-9, and salts thereof.

As used herein, the term « *H1-receptor blocking antihistamine* », or « *H1 antagonist* » or « *H1 blocker* » is meant to refer to a compound capable of blocking the action of histamine at the H1 receptor, which may thus encompasss H1-antihistamines, including those selected from : Loratidine, Cetirizine, Cyporheptadine, Chlorpheniramine, Clemastine, and salts thereof.

As used herein, the term « *essential fatty acid* » refers to the those fatty acids which are considered as essential in Felidae (e.g. cats), so termed because they cannot be synthesized from non-fat sources such as carbohydrate or protein. Essential fatty acids in Felidae (e.g. cats) comprise linoleic acid, linolenic acid and arachidonic acid.

As used herein, the term "feline" or "Felidae" refers to an animal that is a member of the family Felidae; including without limitation the subfamilies, Felinae, Pantherinae, and Acinonychinae; the genera Caracal, Catopuma, Felis, Herpailurus, Leopardus, Leptailurus, Lynx, Oncifelis, Oreailurus, Otocolobus, Prionailurus, Profelis, Puma, Neofelis, Panthera, Pardofelis, and Uncia; the species felis, lybica, jubatus, caracal, badia, bieti, chaus, margarita, nigripes, silvestris, gordonii, yaguarondi, pardalis, tigrinus, wiedi, serval, canadensis, lynx, pardinus, rufus, colocolo, geoffroyi, guigna, jacobita, manul, bengalensis, planiceps, rubiginosus, viverrinus, aurata, concolor, nebulosa, leo, onca, pardus, tigris, marmorata, and uncial. The term may thus include animals, for example companion (or *"pet"*) animals, selected from, without limitation, cheetah, puma, jaguar, leopard, lion, lynx, liger, tiger, panther, bobcat, ocelot, smilodon, caracal, serval and cats. As used herein, cats encompass wild cats and domestic cats, and most preferably domestic cats.

The present disclosure relates, in particular, to domestic cat breed. Domestic cat breeds which are particularly considered, in the context of the present disclosure, may comprise or consist of those recognized by the International Cat Association (TICA). Examples of cat breeds which are considered herein include those selected from the list consisting of: Abyssinian, Aegean, American Bobtail, American Curl, American Ringtail, American Shorthair, American Wirehair, Aphrodite Giant, Arabian Mau, Asian, Asian Semi-longhair, Australian Mist, Balinese, Bambino, Bengal, Birman, Bombay, Brazilian Shorthair, British Longhair, British Shorthair, Burmese, Burmilla, California Spangled, Chantilly-Tiffany, Chartreux, Chausie, Colorpoint Shorthair, Cornish Rex, Cymric, Manx Longhair, Long-haired Manx, Cyprus, Devon Rex, Donskoy, Don Sphynx, Dragon Li, Chinese Li Hua, Dwelf, Egyptian Mau, European Shorthair, Exotic Shorthair, Foldex, German Rex, Havana Brown, Highlander, Himalayan, Colorpoint Persian, Japanese Bobtail, Javanese, Colorpoint Longhair, Kanaani, Khao Manee, Kinkalow, Korat, Korean Bobtail, Korn Ja, Kurilian Bobtail, Kuril Islands Bobtail, Lambkin, LaPerm, Lykoi, Maine Coon, Manx, Mekong Bobtail, Minskin, Minuet, Munchkin, Nebelung, Norwegian Forest Cat, Ocicat, Ojos Azules, Oregon Rex, Oriental Bicolor, Oriental Longhair, Oriental Shorthair, Persian, Peterbald, Pixie-bob, Ragamuffin, Liebling, Ragdoll, Raas, Russian Blue, Russian White, Russian Black, Russian Tabby, Sam Sawet, Savannah, Scottish Fold, Selkirk Rex, Serengeti, Serrade Petit, Siamese, Siberian, Siberian Forest Cat, Neva Masquerade, Singapura, Snowshoe, Sokoke, Somali, Sphynx, Suphalak, Thai, Thai Lilac,Thai Blue Point, Thai Lilac Point, Tonkinese, Toybob, Toyger, Turkish Angora, Turkish Van, Turkish Vankedisi, Ukrainian Levkoy, York Chocolate.

As used herein, the term "food product" or "food composition" or "diet" or "foodstuff" may refer to, for example, without limitation foodstuff, diet, food supplement, liquid and/or a material that may contain proteins, carbohydrates and/or crude fats. For example, the term may also refer to supplementary substances or additives, for example, minerals, vitamins, and condiments (See Merriam- Webster's Collegiate Dictionary, 10th Edition, 1993). Such food compositions or products may be nutritionally complete or not.

As used herein, the term "gastrointestinal sample" may refer to any sample, or fraction thereof, which is susceptible to be derived from the gastrointestinal tract, including the digestive tract as a whole, from the mouth to the anus, which may thus include the higher gastrointernal tract, the lower gastrointestinal tract, and combinations thereof; which may thus include any biological sample or fraction thereof, derived from the mouth, the stomach, the small intestine, the colon, the cecum, the rectum or the anus. A gastrointestinal sample according to the present disclosure may thus comprise, or consist of, a gastric, colorectal, duodenal or rectal sample, or fractions thereof.

As used herein, the terms « fecal sample », « stool sample » and « feces » may be used interchangeably.

A suitable nucleic acid sample, for example a suitable nucleic acid gastrointestinal sample, may thus comprise, or consist of, a nucleic acid sample obtained from a fecal sample, or fractions thereof. Fecal samples are commonly used in the art to sample gut microbiota. A preferred nucleic acid sample may be a nucleic acid sample obtained from a suitable fecal sample.

Methods for obtaining a fecal sample from a subject are known in the art and include, but are not limited to, rectal swab, stool collection, and sampling of the floor or environment where animals defecate {e.g. a pen in a commercial animal farm). Suitable fecal samples may be freshly obtained or may have been stored under appropriate temperatures and conditions known in the art.

As used herein, the term "gastrointestinal sample" may thus encompass a nucleic acid sample (e.g. a nucleic acid fecal sample) which comprises one or more (« a plurality of ») heterogeneous nucleic acids produced by a subject's gut microbiota.

A gastrointestinal sample according to the present disclosure may comprise, or consist of, a stool sample or fractions thereof.

Suitable gastrointestinal samples for interrogation using the methods of the application include but are not limited to: samples of gut contents and/or mucosal biopsies obtained directly by an invasive technique e.g. by surgery, by rectal or intestinal sampling via colonoscopy-type procedures, or by other means. Most preferably, samples are obtained by less invasive methods, e.g. stool samples.

Methods for extracting nucleic acids from a gastrointestinal sample (e.g. a fecal/stool sample) are also well known in the art. The nucleic acids comprising the nucleic acid sample may or may not be amplified prior to being used as an input, depending upon the type and sensitivity of the data acquisition component. When amplification is desired, nucleic acids may be amplified via polymerase chain reaction (PCR) from a nucleic acid sample. Methods for performing PCR are well known in the art. Selection of nucleic acids or regions of nucleic acids to amplify are discussed above. The nucleic acids comprising the nucleic acid sample may also be fluorescently or chemically labeled, fragmented, or otherwise modified prior to sequencing or hybridization to an array as is routinely performed in the art.

As used herein, the term "microbiome" refers to the collection of genomes from all microorganisms, including bacterial, viruses and fungi; in particular to the totality of bacteria, their genetic elements (genomes) in a defined environment, e.g. within the gut of a host, the latter then being referred to as the "gut microbiome". A gut microbiome profile may thus represent the presence, absence or the abundance of one or more of bacterial taxa identified in the gut biological sample; bacterial metabolic products in said gut biological sample; proteins in said gut biological sample ; nucleic acids in said gut biological sample.

Microbial taxa may be defined at any taxonomic level, including phyla, class, order, family, genus, species, strain, or a combination thereof. A skilled artisan will appreciate that while more resolved levels of taxonomy (e.g. genus, species or strain) may generally be more predictive, there may be circumstances where use of higher levels of taxonomy improves the performance of the system.

According to the present disclosure, the said bacteria, bacterial metabolic products and said proteins and/or said nucleic acids are derived from *Helicobacter canis* and/or *Bacteroides vulgatus.* According to some preferred embodiments, the gut microbiome profile can be determined based on an analysis of amplification products of nucleic acids, in particular of DNA and/or RNA of the gut microbiota, e.g. based on an analysis of amplification products of genes coding for one or more of small subunit rRNA, etc. and/or based on an analysis of proteins and/or metabolic products present in the biological sample (e.g. the gastrointestinal sample=. Gut microbiome profiles may be "compared" by any of a variety of statistical analytic procedures).

As used herein, the term "Operational taxonomic unit" ("OTU", plural OTUs)" refers to a terminal leaf in a phylogenetic tree and is defined by a specific genetic sequence and all sequences that share sequence identity to this sequence at the level of species. A "type" or a plurality of "types" of bacteria includes an OTU or a plurality of different OTUs, and also encompasses a strain, species, genus, family or order of bacteria. The specific genetic sequence may be the 16S rRNA sequence or a portion of the 16S rRNA sequence or it may be a functionally conserved housekeeping gene found broadly across the eubacterial kingdom. OTUs typically share at least 95%, 96%, 97%, 98%, or 99% sequence identity ; preferably at least 97% sequence identity. OTUs are frequently defined by comparing sequences between organisms. Sequences with less than 95% sequence identity are generally not considered to form part of the same OTU.

As used herein, a "16S rRNA" may be either the 16S rRNA as such or the genomic sequence from which originates the corresponding 16S rRNA. A 16S rRNA may be sequenced by using primers/probes targeting the V3-V4 hypervariable region, such as DNA or RNA probes, for example DNA probes of sequence **SEQ ID N°1** and **SEQ ID N°2**.

A suitable nucleic acid used for taxonomic classification is universally distributed among the gut microbial population being queried allowing for the analysis. According to some embodiments, the nucleic acid has both a conserved region and at least one region subject to variation. The presence of at least one variable region allows sufficient diversification to provide a tool for classification, while the presence of conserved regions enables the design of suitable primers for amplification (if needed) and/or probes for hybridization for various taxa at different taxonomic levels ranging from individual strains to whole phyla. While any suitable nucleic acid known in the art may be used, one skilled in the art will appreciate that selection of a nucleic acid or region of a nucleic acid to amplify may differ by environment. In some embodiments, a nucleic acid queried is a small subunit ribosomal RNA gene. For bacterial populations, the V regions of the 16s rRNA gene, in particular the V3 and/or V4 regions of the 16s rRNA gene are suitable, though other suitable regions are known in the art. Guidance for selecting a suitable 16S rRNA region to amplify can be found throughout the art, including Guo F et al. PLOS One 8(10) e76185, 2013; Soergel DAW et al. ISME Journal 6: 1440, 2012; and Hamady M et al. Genome Res. 19: 1 141, 2009. For example, a 16S rRNA of the disclosure may be a partial 16S rRNA nucleic acid sequence or a full length 16S rRNA nucleic acid sequence.

When the determination of a level of bacterias (e.g. *helicobacter canis* and/or *bacteroides vulgatus*) is achieved through determination of a nucleic acid, for example of DNA and/or RNA from the one or more gut microbiota, it is preferably achieved through determination of one or more regions, in particular V regions, of the 16S rRNA from said one or more bacteria, and/or the determination of one or more OTUs from the said bacteria.

Suitable nucleic acids used for classification may include a nucleic acid that encodes a polypeptide which can be assigned to a functional group known in the art. The current technology is not limited to any one classification scheme

As used herein, the *"percentage identity"* or *"sequence identity"* between two sequences of proteins or nucleic acids (depending on what is applicable) means the percentage of identical amino acid or base, e.g. nucleotides or nucleosides, residues between the two sequences to be compared, obtained after optimal alignment, this percentage being purely statistical and the differences between the two sequences being distributed randomly along their length. The comparison of sequences is traditionally carried out by comparing the sequences after having optimally aligned them, said comparison being able to be conducted by segment or by using an "alignment window". Optimal alignment of the sequences for comparison can be carried out, in addition to comparison by hand, by means of the local homology algorithm of Smith and Waterman (1981), by means of the local homology algorithm of Neddleman and Wunsch (1970), by means of the similarity search method of Pearson and Lipman (1988) or by means of computer software using these algorithms (GAP, BESTFIT, FASTA and TFASTA in the Wisconsin Genetics Software Package, Genetics Computer Group, 575 Science Dr., Madison, WI, or by the comparison software BLAST NR or BLAST P).

The percentage identity between two sequences is determined by comparing the two optimally-aligned sequences in which the sequence to compare can have additions or deletions compared to the reference sequence for optimal alignment between the two sequences. Percentage identity is calculated by determining the number of positions at which the amino acid residue is identical between the two sequences, preferably between the two complete sequences, dividing the number of identical positions by the total number of positions in the alignment window and multiplying the result by 100 to obtain the percentage identity between the two sequences.

After a sample is obtained, the types and/or the quantity (e.g. occurrence or abundance) in the sample of at least one bacteria of interest (e.g. *Helicobacter canis* and/or *Bacteroides vulgatus)* is determined according to any method known to those of skill in the art. In addition, a total amount of bacteria may be determined, and then for each constituent bacteria, a fractional percentage (e.g. relative amount, ratio, distribution, frequency, percentage, etc.) of the total is calculated. The result is typically correlated with at least one suitable control result, e.g. control results of the same parameter(s) obtained from asymptomatic individuals/Felidae (negative control), and/or individuals/Felidae known to have an inflammatory disorder of interests (positive control), or from individuals/Felidae who have had the inflammatory disorder of interest and are being or have been treated, either successfully or unsuccessfully.

As used herein, a modulation of the level of *Bacteroides vulgatus* or *Helicobacter canis* for a given subject/individual is determined based on a reference value ; which may correspond to an increase or a decrease based on said reference value.

As used herein, "an increase of the level of *Bacteroides vulgatus"* or "an increase of the level of *Helicobacter canis"* over a reference value may encompass any statistically significant increase over said reference value. It will be readily understood herein that the level of increase will thus be necessarily dependent upon the method of detection and of the reference value which is considered. For example, an increase of the level of a reference value may thus be of at least 10%, at least 20%, at least 30%, at least 40%, at least 50%, at least 60%, at least 70%, at least 80%, at least 90%, at least 100% higher or at least 2-fold, at least 3-fold, at least 4-fold, at least 5-fold higher, at least 6-fold higher, at least 7-fold higher, at least 8-fold higher, at least 9-fold higher, at least 10-fold higher, at least 100-fold higher than said reference value.

As used herein, "a decrease of the level of *Bacteroides vulgatus"* or "a decrease of the level of *Helicobacter canis"* below a reference value may encompass any statistically significant increase below said reference value. It will be readily understood herein that the level of decrease will thus be necessarily dependent upon the method of detection and of the reference value which is considered. For example, a decrease of the level of a reference value may thus be of at least 10%, at least 20%, at least 30%, at least 40%, at least 50%, at least 60%, at least 70%, at least 80%, at least 90%, at least 100% lower or at least 2-fold, at least 3-fold, at least 4-fold, at least 5-fold, at least 6-fold, at least 7-fold, at least 8-fold, at least 9-fold, and at least 10-fold lower, at least 100-fold lower than said reference value.

### EXAMPLES

### Material & Methods

### Study population

Animals were recruited in France by four ECVD veterinarians and three (Fr)CertAVP(VD) veterinarians. Twenty feline referral cases presented to the clinic with a feline atopic skin syndrome were enrolled. Clinical signs were consistent with miliary dermatitis (MD) and/or self-inflicted alopecia/hypotrichosis (SIAH) and/or head and neck pruritus (HNP) and/or eosinophilic granuloma complex (EGC). To be included in the study, the patient must be more than 9 months and less than 12 years old, controlled with appropriate external antiparasitic treatment against flea and dewormed. No visible and cytologic signs suggestive of active bacterial and yeast infection was accepted. No diarrhea is also required and no drug intake is tolerated within the last 14 days previous to consultation and sampling. Twenty one healthy cats were recruited for the control group. These cats belonged to the same range of age and were up to date with the usual regular antiparasitic treatments. Their status is free of any clinical sign of disease and are neither suffering of any systemic chronic disease nor receiving any drug. Cats with any signs of skin disease were excluded from the recruitment.

### Sample collection and DNA extraction

Samples were collected from FASS and healthy cats using a sterile swab provided along with a clear tube (Isohelix DAN buccal Swabs, Harrietsham, Kent, UK, reference SK-1S). Each swab was pre-moistened with sterile normal saline solution before being softly introduced into the rectum until the entire collection part disappeared through the anus. The swab was then rotated ten times before being withdrawn. Each swab extremity was stored in a sterile Eppendorf tube and labelled with permanent marker. The tube was frozen at -20°C and kept at the clinic until shipment to a -80°C storage before analysis. The rectal swabs were extracted with the Macherey Nagel Soil Kit using the combination of Lysis Buffer SL1 and Enhancer SX. DNA quantity and quality was assessed before analyzing, and stored at -20°C.

### Amplification and sequencing

In order to assess the bacterial microbiota, two bacterial analysis were performed:
- the Axiom Microbiome Array, using Applied Biosystems^{™} Axiom^{™} assay biochemistry, interrogates nonpolymorphic sequences in both family-conserved and target-specific regions from NCBI database sequences. It detects over 12,000 species, including archaea, bacteria, fungi, protozoa, and viruses. For RNA samples, a reverse transcription reaction was performed with Superscript VILO per the guidance in the Axiom Microbiome Array User Guide. cDNA derived from the samples were used as a substrate for the Axiom whole genome amplification. No DNase treatment was employed on the RNA samples, therefore any DNA from the total nucleic acid preparations will be carried over in the whole genome amplification. The array was hybridized, washed and scanned on the GeneTitan Multi-Channel instrument in an automated fashion according to manufacturer's instructions (Thermo Fisher, Waltham, MA). The 96-well plates were used to run the various clinical and spiked samples to evaluate the array's sensitivity and applicability in clinical samples, as reported in Thissen et al. (« Axiom Microbiome Array, the next generation microarray for high-throughput pathogen and microbiome analysis ». PLoS ONE. 2019).
- PCR amplification of the V3-V4 hypervariable region of the 16S rRNA gene. Library preparation was performed according to the standard instructions of the 16S Metagenomic Sequencing Library Preparation protocol (IlluminaTM, Inc., San Diego, CA, United States). One hypervariable region of the bacterial 16S rRNA gene (the V3-V4 region) was amplified using aliquots of the isolated DNA from each sample. The V3-V4 region of 16S rRNA gene was amplified in each sample by targeting the V3-V4 hypervariable region using the following primers "forward" (TACGGGAGGCAGCAG) **(SEQ ID N°1)** and "reverse" (CCAGGGTATCTAATCC) **(SEQ ID N°2).** The resulting amplicons (456 bp) were subsequently subjected to high-throughput sequencing using the Illumina MiSeq platform. All low quality (<Q20) terminal bases were removed using Trimmomatic (v0.35), thus retaining the high quality reads only. All sequencing results after quality control, which are used in the project along with the metadata, have been submitted to Sequence Read Archive (SRA) NCBI database (accession number PRJNA554535).

### Microarray data analysis.

Microarray data were analyzed using the MiDAS software (Axiom Microbial Detection Analysis Software) (Thermo Fisher) which is based on the Composite Likelihood Maximization Method (CLiMax) algorithm developed at LLNL. Axiom MiDAS performs single-sample analysis of CEL files from Axiom Microbiome Arrays and automatically generates a comprehensive analysis summary in a simple-to-use software package. Probes with signal intensity above the 99th percentile of the random control probe intensities and with more than 20% of target-specific probes detected were considered positive. MiDAS uses initial and conditional scores to determine the likelihood of the target presence. The initial score is the log likelihood ratio for the target being present in the sample if no other targets are present, vs no targets being present in the sample. This value gives information on what the maximum possible contribution of that target is to the holistic model of the sample, based on the probes observed when interrogating a sample with the Axiom Microbiome Array. The conditional score gives an indicator of the actual contribution of each target to the model of the sample; it is the log likelihood for a model including the target vs for a model without the target. As the conditional score takes into account the presence of other targets, it can be lower than the initial score for a given target if there are probes in common between targets.

### 16S data analysis.

The filtered high-quality reads were stitched by using FLASH (v1.2.11). Script multiple_join_paired_ends.py of QIIME (1.8.0) was used to assemble the 16S rRNA amplicons. Moreover, mismatched barcodes and sequences with length less than the threshold (200 bases), were removed. Chimeric sequences were removed using UCHIME (v4.2) providing valid tags (a high-quality sequence) for downstream microbial diversity analysis. These quality-filtered reads were clustered into operational taxonomic units (OTUs; 97% identity) using de novo OTU picking and taxonomic assignment by implementing VSEARCH (v2.4.2) against Silva (v123), and Greengenes (gg13.8) reference databases utilizing QIIME script (pick_de_novo_ otus.py). The representative sequences were classified and annotated by the Naive Bayesian classification algorithm of RDP classifier (v2.2) as reported in Ahmad et al. (« Analysis of gut microbiota of obese individuals with type 2 diabetes and healthy individuals » ; PLoS ONE. 2019).

### Results

The main goal of this study is to compare the gut microbiota of healthy cats and cats with feline atopic skin syndrome (FASS), in order to establish if the loss of diversity associated with atopic status in human and dogs is also present in cats; and, if applicable, to identify significant gut microbiota variations between the two groups. The two final groups (healthy vs. atopic/allergic or FASS) identified for the study are reported hereafter.

**Table 1: Patient characteristic analysis**

| | **FASS (N=14)** | **Healthy (N=12)** | **Unadjusted p value** |
|---|---|---|---|
| **Age, median in years** | 4.50 | 3.00 | 0.054 |
| **Sex, number of Female (%)** | 11 (79%) | 7 (58%) | 0.401 |
| **Life, number of Indoor(%)** | 5 (36%) | 6 (50%) | 0.692 |
| **Neutered, number of (%)** | 14 (100%) | 6 (50%) | 0.004 |
| **Breed, number (%)** | | | 0.261 |
| - Abyssain | 1 (7%) | 0 (0%) | |
| - BLH | 1 (7%) | 0 (0%) | |
| - DSH | 12 (68%) | 8 (67%) | |
| - Main Coon | 0 (0%) | 1 (8%) | |
| - Savannah | 0 (0%) | 1 (8%) | |
| - Siamois | 0 (0%) | 1 (8%) | |
| - Syberian | 0 (0%) | 1 (8%) | |

The table reports the main characteristics from the two groups. No strong differences were observed, with an exception for neutered, when only 50% of cats were neutered from the control group. This ratio imbalance is correlated with healthy cat recruitments when neutering. **Deusch et al.** ("A Longitudinal Study of the Feline Faecal Microbiome Identifies Changes into Early Adulthood Irrespective of Sexual Development; PLOS ONE; 2015) has demonstrated that feline gut microbiota is quite stable since 9 months of age (the youngest in our study was 9 months old). Also, it has been demonstrated that neither gender, neutered nor age when neutered impact gut microbiota. Yet, no statistical difference was noticed in bacterial proportions between neutered and unspayed cats.

Accordingly, it is found that the two groups are comparable for further studies.

Based on statistical analysis of the microarray assay, it is found that among the 30 most frequently identified bacterial strains, the prevalence of *Helicobacter canis* and *Bacteroides vulgatus* varies significantly between the two groups (healthy vs. allergic).

**Table 2: modulation of two species in Healthy vs. FASS groups**

| | **FASS (N=14)** | **Healthy (N=12)** | **Unadjusted p value** |
|---|---|---|---|
| *Bacteroides vulgatus* | 13 (93%) | 6 (50%) | 0.026 |
| *Helicobacter canis* | 2 (14%) | 7 (58%) | 0.038 |

It is found that is *Helicobacter canis* is not present in 60% of healthy cats and present in 14% of FASS (unadjusted p-value: 0.038= statistically significant).

It is found that *Bacteroides vulgatus* is not present in 50% of healthy cats and present in nearly 100% of FASS (unadjusted p-value: 0.026= statistically significant).

## Claims

1. An *in vitro* method for the diagnosis or follow-up of an inflammatory disorder in a Felidae, comprising a step of determining the level of occurrence of *Bacteroides vulgatus* or *Helicobacter canis* in a digestive tract sample thereof; wherein:
- an increase of the level of *Bacteroides vulgatus* is indicative of the occurrence of the inflammatory disorder, and/or
- a decrease of the level of *Helicobacter canis* is indicative of the occurrence of the inflammatory disorder, and/or
- an increase of the ratio level of *Bacteroides vulgatus* over *Helicobacter canis,* is/are indicative of the inflammatory disorder.

2. The *in vitro* method according to claim 1, comprising a step of determining the level of occurrence of *Bacteroides vulgatus* in the digestive tract sample thereof.

3. The *in vitro* method according to claim 1, comprising a step of determining the level of occurrence of *Helicobacter canis* in the digestive tract sample thereof.

4. The *in vitro* method according to claim 1, comprising a step of determining the level of occurrence of *Bacteroides vulgatus* and *Helicobacter canis* in the digestive tract sample thereof.

5. The *in vitro* method according to any of the preceding claims, wherein the inflammatory disorder is selected from a list consisting of: an allergic disorder, an inflammatory skin disorder, an inflammatory tract disorder, and a respiratory tract disorder.

6. The *in vitro* method according to any of the preceding claims, wherein the inflammatory disorder is Feline atopic syndrome (FAS).

7. The *in vitro* method according to any of the preceding claims, wherein the inflammatory disorder is selected from: Feline atopic skin syndrome (FASS), Flea allergy dermatitis (FAD), Feline food allergy (FFA).

8. The *in vitro* method according to any of the preceding claims, wherein the inflammatory disorder is Feline atopic skin syndrome (FASS).

9. The *in vitro* method according to any of the preceding claims, wherein the inflammatory disorder is selected from: miliary dermatitis (MD), self-inflicted alopecia/hypotrichosis (SIAH), head and neck pruritus (HNP), eosinophilic granuloma complex (EGC).

10. The *in vitro* method according to any of the preceding claims, wherein the digestive tract sample is selected from a list consisting of: a fecal sample, a gastric sample, a saliva sample, or fractions thereof; in particular a fecal sample or fractions thereof.

11. The *in vitro* method according to any of the preceding claims, wherein the Felidae is selected from a list consisting of: cheetah, puma, jaguar, leopard, lion, lynx, liger, tiger, panther, bobcat, ocelot, smilodon, caracal, serval and cats; in particular wild cats and domestic cats, including breeds and hybrids thereof; and most preferably domestic cats.

12. The *in vitro* method according to any of the preceding claims, wherein the Felidae is selected from a list consisting of: Abyssinian, Aegean, American Bobtail, American Curl, American Ringtail, American Shorthair, American Wirehair, Aphrodite Giant, Arabian Mau, Asian, Asian Semi-longhair, Australian Mist, Balinese, Bambino, Bengal, Birman, Bombay, Brazilian Shorthair, British Longhair, British Shorthair, Burmese, Burmilla, California Spangled, Chantilly-Tiffany, Chartreux, Chausie, Colorpoint Shorthair, Cornish Rex, Cymric, Manx Longhair, Long-haired Manx, Cyprus, Devon Rex, Donskoy, Don Sphynx, Dragon Li, Chinese Li Hua, Dwelf, Egyptian Mau, European Shorthair, Exotic Shorthair, Foldex, German Rex, Havana Brown, Highlander, Himalayan, Colorpoint Persian, Japanese Bobtail, Javanese, Colorpoint Longhair, Kanaani, Khao Manee, Kinkalow, Korat, Korean Bobtail, Korn Ja, Kurilian Bobtail, Kuril Islands Bobtail, Lambkin, LaPerm, Lykoi, Maine Coon, Manx, Mekong Bobtail, Minskin, Minuet, Munchkin, Nebelung, Norwegian Forest Cat, Ocicat, Ojos Azules, Oregon Rex, Oriental Bicolor, Oriental Longhair, Oriental Shorthair, Persian, Peterbald, Pixie-bob, Ragamuffin, Liebling, Ragdoll, Raas, Russian Blue, Russian White, Russian Black, Russian Tabby, Sam Sawet, Savannah, Scottish Fold, Selkirk Rex, Serengeti, Serrade Petit, Siamese, Siberian, Siberian Forest Cat, Neva Masquerade, Singapura, Snowshoe, Sokoke, Somali, Sphynx, Suphalak, Thai, Thai Lilac,Thai Blue Point, Thai Lilac Point, Tonkinese, Toybob, Toyger, Turkish Angora, Turkish Van, Turkish Vankedisi, Ukrainian Levkoy, York Chocolate.

13. The *in vitro* method according to any of the preceding claims, wherein the step of determining the level of occurrence of *Bacteroides vulgatus* or *Helicobacter canis* comprises detecting, preferably quantifying, all or part of a nucleic acid sequence of said *Bacteroides vulgatus* **or** *Helicobacter canis.*

14. A compound, or composition thereof, selected from the list consisting of:
- a corticosteroid, in particular a glucocorticoid or a mineralocorticosteroid;
- ciclosporin;
- oclatinib;
- HI-receptor blocking antihistamine ;
- essential fatty acid selected from the group consisting of: linoleic acid, linolenic acid and arachidonic acid ;
- palmitoylethanolamide (PEA)
- and combinations thereof;
for use in a method for treating an inflammatory disorder in a Felidae, said Felidae being **characterized by**: an increased level of *Bacteroides vulgatus* in the digestive tract, a decreased level of *Helicobacter canis* in the digestive tract, and/or an increased ratio level of *Bacteroides vulgatus* over *Helicobacter canis* in the digestive tract.

15. The compound, or composition thereof, according to the preceding claim; for use in a method for treating an inflammatory disorder in a Felidae, said inflammatory disorder being Feline atopic skin syndrome (FASS).
